# EUROPEAN PATENT SPECIFICATION

(11) **EP 2 963 612 B1**
(45) Date of publication and mention of the grant of the patent: **24.06.2020**
(21) Application number: 13876421.2
(22) Date of filing: 11.12.2013
(51) Int. Cl.: G16H 20/60, G06Q 50/22, G06Q 10/00

(54) **INFORMATION PROCESSING DEVICE AND STORAGE MEDIUM**
INFORMATIONSVERARBEITUNGSVORRICHTUNG UND SPEICHERMEDIUM
DISPOSITIF DE TRAITEMENT D'INFORMATIONS ET SUPPORT DE STOCKAGE

(30) Priority: 28.02.2013 JP 2013039356
(43) Date of publication of application: 06.01.2016
(73) Proprietor: Sony Corporation, Tokyo 108-0075 (JP)
(72) Inventor: SAKO, Yoichiro, Tokyo 108-0075 (JP); HANAYA, Hiroyuki, Tokyo 108-0075 (JP); KAMADA, Yasunori, Tokyo 108-0075 (JP); KOGA, Yuki, Tokyo 108-0075 (JP); ONUMA, Tomoya, Tokyo 108-0075 (JP); TAKEHARA, Mitsuru, Tokyo 108-0075 (JP); NAKAMURA, Takatoshi, Tokyo 108-0075 (JP); HAYASHI, Kazunori, Tokyo 108-0075 (JP); KON, Takayasu, Tokyo 108-0075 (JP); TANGE, Akira, Tokyo 108-0075 (JP)
(74) Representative: MFG Patentanwälte Meyer-Wildhagen Meggle-Freund Gerhard PartG mbB
(86) International application number: PCT/JP2013/083229
(87) International publication number: WO 2014/132521

(56) References cited:
- WO-A1-2012/115297
- WO-A2-2012/170587
- JP-A- 2011 058 782
- JP-A- 2011 107 768
- JP-A- 2011 118 683
- JP-A- 2011 210 119
- JP-A- 2012 238 106
- JP-A- 2013 114 315
- US-A1- 2012 179 665

## Description

### Technical Field

The present disclosure relates to an information processing device and a storage medium.

### Background Art

Recently, devices that assist dietary lifestyle management are being proposed.

Further, Patent Literature 2 below discloses technology that reduces the user workload of recording meal content for efficient management. Specifically, if a food image is sent together with time and date information from a personal client to a center server, an advisor (expert) at the center server analyzes the image of food, and inputs and sends advice.

Also, Patent Literature 2 below discloses a navigation system that includes a nutritional data storage unit (for example, an IC tag attached to a packaging container of a food product), a nutritional data reading unit (for example, an IC tag reader), and a data output unit, which reduces the long time and effort required to memorize food composition.

Patent Literature 3 below relates to a health monitoring system that can include an intake tracker, an output tracker, a personal monitor, a recommender, a product ID and nutrition database, a personal database, an input, and a display. The health monitoring system can track the food that a user eats, the exercise that a user does, and the user's personal characteristics in order to provide health assessments and recommendations. For example, the health monitoring system can be used to scan the barcode of a food product, take a picture of the food product packaging, take a picture of the food itself, or manually record the food item. The images can be matched with images in a database in order to associate nutritional or other information with the food being consumed.

Further, Patent Literature 4 below discloses a device comprising a nutrition manager configured to receive data representing parameters relating to nutrition intake of a user and to maintain data representing one or more nutrition profiles. Nutrition-related parameters describe characteristics, factors or attributes of consumable materials (e.g., food and drink), including nutrients, such as vitamins, minerals, etc. that a user consumes. Nutrition-related parameters also include calories. The nutrition-related parameters can be formed from sensor data or derived based on computations.

Still further, Patent Literature 5 below discloses data analysis of a food item based on one or more digital images of the food item.

### Citation List

### Patent Literature

Patent Literature 1: JP 2010-33326A
Patent Literature 2: JP 2006-195943A
Patent Literature 3: US 2012/179665 A1
Patent Literature 4: WO 2012/170587 A2
Patent Literature 5: WO 2012/115297 A1

### Summary of Invention

### Technical Problem

However, with Patent Literature 1 above, since analysis is conducted on the basis of a captured image of an already-prepared dish, it is difficult to recognize what kinds of ingredients and how much were used in the cooking process.

Also, Patent Literature 2 likewise does not discuss the computation of calories or nutritional components during the cooking process.

### Description

### Summary of Invention

### Technical Problem

However, with Patent Literature 1 above, since analysis is conducted on the basis of a captured image of an already-prepared dish, it is difficult to recognize what kinds of ingredients and how much were used in the cooking process.

Also, Patent Literature 2 likewise does not discuss the computation of calories or nutritional components during the cooking process.

Accordingly, the present disclosure proposes a new and improved information processing device and storage medium capable of computing indices during a cooking process.

### Solution to Problem

An information processing device and a storage medium as defined in the appended claims are disclosed.

According to the present disclosure, there is provided an information processing device including: an estimation unit configured to estimate a used quantity of at least one of an ingredient to be cooked and a seasoning used in cooking, on the basis of a signal detected by a ; sensor in real-time every time an ingredient or seasoning is added during a cooking process; an index computation unit configured to compute a cooking index indicating a value of mass and/or a calorific value of the ingredient or the seasoning according to the estimated quantity of the ingredient or the seasoning, by accumulating cooking indices computed since the cooking process started to a cumulative cooking index; and a notification control unit configured to perform control to issue a notification of the cooking index computed by the index computation unit.

According to the present disclosure, there is provided a storage medium having a program stored therein, the program causing a computer to function as: an estimation unit configured to estimate a used quantity of at least one of an ingredient to be cooked and a seasoning used in cooking, on the basis of a signal detected by a sensor in real-time every time an ingredient or seasoning is added during a cooking process; an index computation unit configured to compute a cooking index indicating a value of mass and/or a calorific value of the ingredient or the seasoning according to the estimated quantity of the ingredient or the seasoning, by accumulating cooking indices computed since the cooking process started to a cumulative cooking index; and a notification control unit configured to perform control to issue a notification of the cooking index computed by the index computation unit.

### Advantageous Effects of Invention

According to the present disclosure as described above, it becomes possible to compute indices during a cooking process.

### Brief Description of Drawings

[FIG. 1] FIG. 1 is a diagram for explaining an overview of a cooking indices display according to an embodiment of the present disclosure.
[FIG. 2] FIG. 2 is a block diagram illustrating an exemplary internal configuration of an HMD according to the present embodiment.
[FIG. 3] FIG. 3 is a diagram for explaining a used ingredient/seasoning quantity estimation method by a used quantity estimation unit according to the present embodiment.
[FIG. 4] FIG. 4 is a flowchart illustrating a cooking indices display process according to the present embodiment.
[FIG. 5A] FIG. 5A is a diagram illustrating an example screen display of cooking indices in the case of adding an ingredient.
[FIG. 5B] FIG. 5B is a diagram illustrating an example screen display of cooking indices in the case of adding a seasoning.
[FIG. 6] FIG. 6 is a diagram illustrating an example image display of a warning display according to the present embodiment.
[FIG. 7] FIG. 7 is a diagram illustrating an example screen display of a comparison result notification according to the present embodiment.
[FIG. 8] FIG. 8 is a block diagram illustrating a functional configuration of a main controller when using cooking indices to present consumption indices while eating and drinking.
[FIG. 9] FIG. 9 is a flowchart illustrating an operational process when using cooking indices to notify a user of consumption indices while eating and drinking.

### Description of Embodiments

Hereinafter, (a) preferred embodiment(s) of the present disclosure will be described in detail with reference to the appended drawings. Note that, in this specification and the drawings, elements that have substantially the same function and structure are denoted with the same reference signs, and repeated explanation is omitted.

Hereinafter, the description will proceed in the following order.
1. Overview of cooking indices display according to an embodiment of the present disclosure
2. Basic configuration and operational process of HMD
   2-1. Basic configuration of HMD
   2-2. Operational process of HMD
3. Screen display examples
   3-1. Indices display
   3-2. Display based on comparison result
4. Supplemental remarks
5. Conclusion

### «1. Summary of cooking indices display according to embodiment of present disclosure»

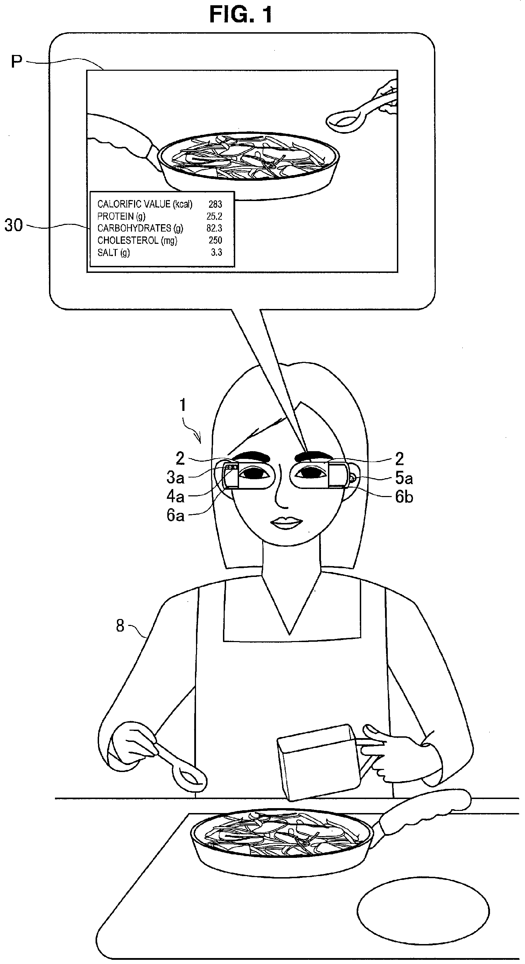

First, a cooking indices display according to an embodiment of the present disclosure will be summarized with reference to FIG. 1.

FIG. 1 is a diagram summarizing a cooking indices display according to an embodiment of the present disclosure. As illustrated in FIG. 1, a user 8 is wearing an eyeglasses-style head-mounted display (HMD) 1. The HMD 1 includes a wearing unit having a frame structure that wraps halfway around the back of the head from either side of the head, for example, and is worn by the user 8 by being placed on the pinna of either ear, as illustrated in FIG. 1.

Also, the HMD 1 is configured such that, in the worn state, a pair of display units 2 for the left eye and the right eye are placed immediately in front of either eye of the user 8, or in other words at the locations where the lenses of ordinary eyeglasses are positioned. A captured image of a real space captured with an image capture lens 3a, for example, is displayed on the display units 2. The display units 2 may also be transparent, and by having the HMD 1 put the display units 2 in a see-through state, or in other words a transparent or semi-transparent state, ordinary activities are not impaired even if the user 8 wears the HMD 1 continuously like eyeglasses.

Also, as illustrated in FIG. 1, in the HMD 1, the image capture lens 3a is placed facing forward, so as to capture the direction in which the user sees as the photographic direction while in a state of being worn by the user 8. Furthermore, a light emitter 4a that provides illumination is provided in the image capture direction by the image capture lens 3a. The light emitter 4a is formed by a light-emitting diode (LED), for example.

Also, although only illustrated on the left eye side in FIG. 1, a pair of earphone speakers 5a which may be inserted into a user's right ear canal and left ear canal in the worn state are provided. Also, microphones 6a and 6b that pick up external sounds are placed to the right of the display unit 2 for the right eye, and to the left of the display unit 2 for the left eye.

Note that the external appearance of the HMD 1 illustrated in FIG. 1 is an example, and that a variety of structures by which a user may wear the HMD 1 are conceivable. It is sufficient for the HMD 1 to be formed as a worn unit of the eyeglasses type or head-mounted type, and at least for the present embodiment, it is sufficient for a display unit 2 to be provided close in front of a user's eye. Also, besides the display units 2 being provided as a pair corresponding to either eye, a configuration providing a single display unit 2 corresponding to an eye on one side is also acceptable.

Also, although the image capture lens 3a and the light emitter 4a that provides illumination are placed facing forward on the side of the right eye in the example illustrated in FIG. 1, the image capture lens 3a and the light emitter 4a may also be placed on the side of the left eye, or placed on both sides.

It is also acceptable to provide a single earphone speaker 5a to be worn in only one ear, rather than as left and right stereo speakers. Likewise, a microphone may be one of either the microphone 6a or 6b.

Furthermore, a configuration not equipped with the microphones 6a and 6b or the earphone speakers 5a is also conceivable. A configuration not provided with the light emitter 4a is also conceivable.

The above thus describes an external configuration of the HMD 1 illustrated in FIG. 1. In the present specification, an HMD 1 is used as an example of an information processing device that conducts a cooking indices display, but an information processing device according to the present disclosure is not limited to an HMD 1. For example, the information processing device may also be a smartphone, a mobile phone, a personal digital assistant (PDA), a personal computer (PC), a tablet device, or the like.

Herein, as discussed earlier, with Patent Literature 1 above, it is difficult to analyze what kinds of ingredients and how much were used in the cooking process on the basis of a captured image of an already-prepared dish. Also, if cooking indices indicating the calories and mass of ingredients and seasonings could be computed in real-time during the cooking process and including in a notification to the user, dietary lifestyle support would be possible from the cooking stage.

Particularly, in cases in which improvements in dietary lifestyle are demanded due to problems of lifestyle-related diseases or the like, the intake and numerical values of calories, fat, sugar, purines, cholesterol, and the like become problematic, but there is a large amount of information related to food substances that are preferred and non-preferred for improving dietary lifestyle, which becomes a burden on the user. In addition, it has been difficult for a user to continually take in preferred food substances, as in some cases the user may forget when eating or cooking, or an unexpected food substance may be non-preferred.

Accordingly, focusing on the above circumstances led to the creation of an HMD 1 (information processing device) according to the embodiments of the present disclosure. An HMD 1 according to the embodiments of the present disclosure is able to compute indices during the cooking process.

Specifically, the HMD 1 (information processing device) according to the present embodiment captures a cooking state with an image capture lens 3a, estimates the quantities of used ingredients or used seasonings in the captured image, computes cooking indices of each ingredient or seasoning on the basis of the estimation results, and notifies the user of the computed cooking indices in real-time. In this specification, cooking indices are the values of the mass or calorific value (calories) of ingredients or seasonings, for example. Also, the values of the mass or calorific value may also be the values of the mass or calorific value of respective nutritional components included in ingredients or seasonings. The respective nutritional components may be vitamins, fat, protein, carbohydrates, calcium, magnesium, dietary fiber, potassium, iron, retinol, salt, sugar, purines, cholesterol, or alcohol, for example. In addition, the quantities of used ingredients and used seasonings may be estimated on the basis of not only a captured image, but also results detected from a smell sensor or various concentration sensors (such as a salt sensor or sugar sensor). The various concentration sensors are provided in a cooking utensil such as a measuring spoon or pot, and detected measurement data is transmitted to the HMD 1.

For example, in the example illustrated in FIG. 1, the HMD 1 captures the cooking process of the user 8 with an image capture lens 3a, and through image analysis, recognizes cooking utensils (objects) such as a salt container, measuring spoons (teaspoon/tablespoon), and a frying pan. Cooking utensils may be recognized by not only analysis of a captured image, but also by reading an IC tag, marker, two-dimensional barcode, or the like provided on a cooking utensil. Subsequently, the HMD 1 recognizes an action of the user 8 scooping salt from the salt container with a measuring spoon and adding (or being about to add) the salt to the frying pan, computes the mass of salt as a cooking index and notifies the user of computed index, or accumulates and totals the amount of salt added to the dish and notifies the user of the cumulative amount of salt.

For the method of notifying the user of cooking indices, a display notification by the display units 2 or an audio notification by the earphone speakers 5a is possible. For example, as illustrated in FIG. 1, the HMD 1 notifies the user 8 by displaying on the display units 2 a cooking indices display 30 that indicates the calorific value and mass of each ingredient and seasoning. The HMD 1, by displaying a cooking indices display 30 that is totaled in real-time every time an ingredient or seasoning is added, is able to notify the user 8 of cooking indices during the cooking process. At this point, the HMD 1 may display the cooking indices display 30 superimposed onto a captured image P, or set the display units 2 to semi-transparent and display the cooking indices display 30 in a state in which the scene of the real space is visible.

Additionally, the HMD 1 may also compare a cooking index to a prescribed value, and present a warning notification to the user when the cooking index approaches the prescribed value. A prescribed value refers to a limit index, and specifically is a prescribed value prioritizing health, or a prescribed value prioritizing the user's taste preferences. Additionally, the HMD 1 may also compare a cooking index to a prescribed value for when cooking is complete, and present a warning notification to the user according to the comparison result.

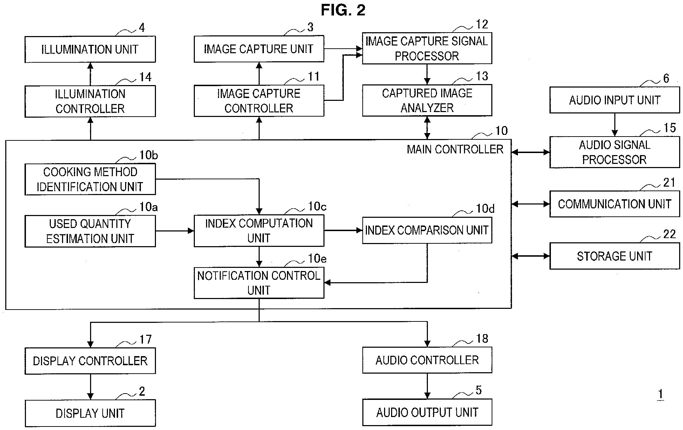

The above thus summarizes a cooking indices display according to the present embodiment. Next, a basic configuration and operational process of an HMD 1 that presents a cooking indices display according to the present embodiment will be described with reference to FIGS. 2 to 4.

### «2. Basic configuration and operational process of HMD»

### <2-1. Basic configuration of HMD>

FIG. 2 is a diagram illustrating an exemplary internal configuration of an HMD 1 according to the present embodiment. As illustrated in FIG. 2, an HMD 1 according to the present embodiment includes display units 2, an image capture unit 3, an illumination unit 4, an audio output unit 5, an audio input unit 6, a main controller 10, an image capture controller 11, an image capture signal processor 12, a captured image analyzer 13, an illumination controller 14, an audio signal processor 15, a display controller 17, an audio controller 18, a communication unit 21, and a storage unit 22.

### (Main controller 10)

The main controller 10 is made up of a microcomputer equipped with a central processing unit (CPU), read-only memory (ROM), random access memory (RAM), non-volatile memory, and an interface unit, for example, and controls the respective components of the HMD 1.

As illustrated in FIG. 2, the main controller 10 according to the present embodiment functions as a used quantity estimation unit 10a, a cooking method identification unit 10b, an index computation unit 10c, an index comparison unit 10d, and a notification control unit 10e.

The used quantity estimation unit 10a estimates the used quantity of at least one of an ingredient to be cooked and a seasoning used in cooking, on the basis of a signal detected by a sensor, and supplies an estimation result to the index computation unit 10c. A signal detected by a sensor refers to information such as a captured image captured by an image capture sensor (image capture unit 3), numerical values detected by various concentration sensors, and smell data detected by a smell sensor, for example.

The various concentration sensors (such as a salt concentration sensor, sugar concentration sensor, ion concentration sensor, and pH sensor) are provided in a cooking utensil such as a measuring spoon or pot, and detected measurement data is transmitted to the HMD 1, as discussed earlier.

In addition, a smell sensor is provided in the HMD 1 or externally (such as in a cooking utensil or in the kitchen), and detects smell components in an ingredient or seasoning. Herein, a smell sensor may be configured using multiple types of metal-oxide-semiconductor sensor elements, for example. Ordinarily, a metal-oxide-semiconductor is in a state of low conductivity, in which oxygen present in the air is adsorbed on the surface of crystal grains, and this oxygen traps electrons in the crystals which are the carriers. In this state, if smell components adhere to the surface of the metal-oxide-semiconductor, oxidation of the smell components takes away adsorbed oxygen on the surface, and the conductivity increases. Since the change in conductivity differs according to differences in the type and grain size of the metal-oxide-semiconductor, and the catalyst to be added, smell components are identified by utilizing this property.

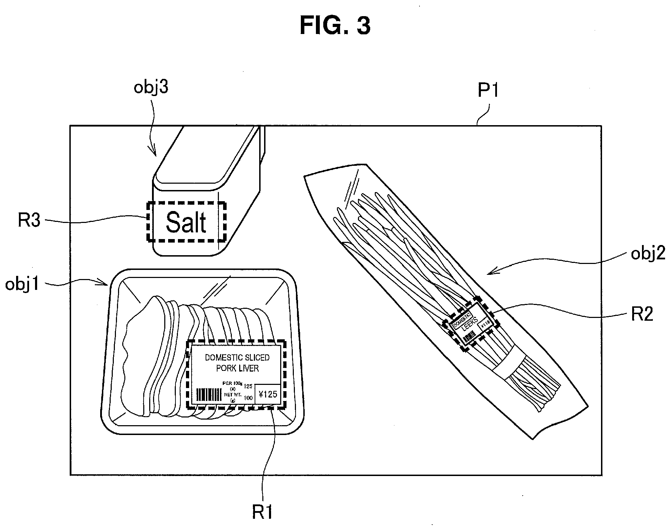

In the case of estimating on the basis of a captured image, the used quantity estimation unit 10a estimates the quantity of ingredients and seasonings used by using an analysis result by the captured image analyzer 13 discussed later. For example, the used quantity estimation unit 10a is able to recognize an ingredient/seasoning on the basis of character recognition or by reading a barcode or marker on a label affixed to a container or bag holding an ingredient or seasoning, and estimate the quantity of recognized ingredient/seasoning used. At this point, an ingredient/seasoning quantity estimation method by the used quantity estimation unit 10a will be described with reference to FIG. 3.

FIG. 3 is a diagram for explaining a used ingredient/seasoning quantity estimation method by the used quantity estimation unit 10a. As illustrated in FIG. 3, when a first ingredient obj1, a second ingredient obj2, and a seasoning container obj3 are extracted from a captured image P1, the used quantity estimation unit 10a conducts character recognition on the character regions R1, R2, and R3 of each object, and conducts ingredient/seasoning recognition and used quantity estimation. In the example illustrated in FIG. 3, the first ingredient obj1 may be recognized as "pork liver", the second ingredient obj2 as "leeks", and the seasoning container obj3 as "salt". Also, in the example illustrated in FIG. 3, the used quantity estimation unit 10a may recognize the characters "Net weight: 100 g" from the character region R1 of the first ingredient obj1, and estimate the quantity of pork liver used.

In addition, the used quantity estimation unit 10a is also able to reference an analysis result of a captured image, and on the basis of features such as the color, shape, and size of the analyzed ingredient/seasoning, recognize an ingredient/seasoning and estimate the quantity of recognized ingredient/seasoning used. In this case, the used quantity estimation unit 10a may also use used quantity estimation data stored in the storage unit 22 (a database associating features such as color, shape, and size with ingredients/seasonings).

Furthermore, the used quantity estimation unit 10a is also able to combine captured image analysis results from the captured image analyzer 13, smell data detected by a smell sensor, and various measurement data to comprehensively estimate the quantity of ingredient/seasoning used.

The cooking method identification unit 10b determines a cooking method during the cooking process (such as stir-fried, grilled, boiled, fried, steamed, raw, or dressed), and supplies a determined result to the index computation unit 10c. Specifically, the cooking method identification unit 10b may identify a cooking method on the basis of a captured image acquired by an image capture sensor (the image capture unit 3), and measurement data detected by sensors such as an infrared sensor (thermal imaging sensor), a temperature sensor, and a humidity sensor. For example, the cooking method identification unit 10b may also identify a cooking method according to a cooking utensil recognized from a captured image (such as a frying pan, pot, tempura pot, or steamer). In addition, the cooking method identification unit 10b may also identify a cooking method in conjunction with various measurement data (such as temperature data and humidity data). In this case, the cooking method identification unit 10b may also use cooking method identification data stored in the storage unit 22 (a database associating cooking utensils, temperature data, and the like with cooking methods).

The index computation unit 10c computes prescribed cooking indices according to the quantity of ingredient/seasoning used that is estimated by the used quantity estimation unit 10a. In addition, the index computation unit 10c is also able to convert computed cooking indices according to the cooking method identified by the cooking method identification unit 10b. The index computation unit 10c supplies computed/converted cooking indices to the index comparison unit 10d and the notification control unit 10e. Also, the computed/converted cooking indices are accumulated in the storage unit 22.

The index computation unit 10c may also compute cooking indices by using index computation data stored in the storage unit 22 (a database associating ingredient/seasoning quantities with cooking indices). For example, the index computation unit 10c computes a teaspoon of salt as "table salt 6 g", and a pinch of salt (the quantity picked up with the three fingers of the thumb, index finger, and middle finger) as "table salt 1 g".

Also, since cooking indices (calories and mass) change according to cooking method in some cases depending on the nutritional components, the index computation unit 10c may also convert computed indices according to a cooking method identified by the cooking method identification unit 10b. For example, the index computation unit 10c converts cooking indices using cooking index conversion data stored in the storage unit 22 (a database associating cooking methods with changes in respective cooking indices).

In addition, the index computation unit 10c is also able to total computed/converted cooking indices and cooking indices accumulated in the storage unit 22.

The index comparison unit 10d compares cooking indices supplied from the index computation unit 10c to prescribed values, and supplies comparison results to the notification control unit 10e. Specifically, the index comparison unit 10d compares a cooking index to a limit index prioritizing health, and judges whether or not the cooking index approaches the limit index (is within a prescribed range based on the limit index). A limit index prioritizing health refers to an upper limit value on the intake of a cooking index that is generally acceptable from a health perspective, or alternatively, an upper limit value on the intake of a cooking index that is acceptable on the basis of information about the user 8 such as medical information (including disease history and medication history), health information (including current physical condition information), genetic information, and predisposition information (including allergy information). Information about the user 8 such as medical information and health information may be extracted from the storage unit 22, or acquired from a designated server via the communication unit 21. In addition, current physical condition information about the user 8 may be detected with various biological sensors that detect biological information (such as blood pressure, body temperature, pulse, or brain waves) provided on the HMD 1, or detected with a biological information detection device (not illustrated) separate from the HMD 1 and possessed by the user.

On the other hand, depending on the preferences of the user 8, a limit index prioritizing health may not necessarily be optimal, and thus the index comparison unit 10d may also compare a cooking index to a limit index prioritizing the preferences of the user 8, and judge whether or not the cooking index approaches the limit index (is within a prescribed range based on the limit index).

In addition, when a limit index (upper limit value) for when cooking is complete may be acquired, the index comparison unit 10d may also compare a cooking index to a limit index (upper limit value) for when cooking is complete. For example, to avoid situations in which the salt content would exceed the upper limit value when adding cheese, dry-cured ham, or a sauce to finish cooking, the HMD 1 notifies the user of the comparison result between the limit index (upper limit value) for when cooking is complete and the current cooking index (a cumulative dish index) during the cooking process. Consequently, the user 8 is able to avoid adding too much salt while cooking. Note that a limit index (upper limit value) for when cooking is complete may be acquired on the basis of recipe data for the dish currently being cooked, or a dish name specified by the user 8.

The notification control unit 10e applies control to notify the user 8 of cooking indices supplied from the index computation unit 10c and comparison results supplied from the index comparison unit 10d. Notification to the user 8 may be a display notification or an audio notification. In the case of a display notification, the notification control unit 10e generates and outputs to the display controller 17 image data indicating cooking indices (for example, the cooking indices display 30 illustrated in FIG. 1), while in the case of an audio notification, the notification control unit 10e generates and outputs to the audio controller 18 an audio signal that vocalizes the cooking indices.

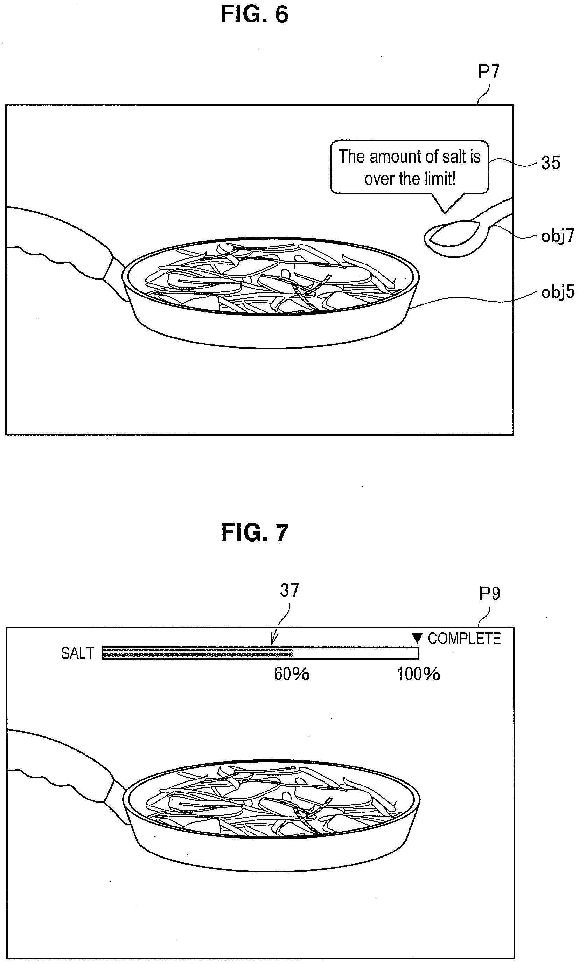

In addition, the notification control unit 10e applies control to present a warning notification when a cooking index approaches a limit index (is within a prescribed range based on the limit index) on the basis of a comparison result supplied from the index comparison unit 10d. A warning notification to the user 8 may be a display notification, an audio notification, a vibration notification, or the like. In the case of a display notification, the notification control unit 10e generates and outputs to the display controller 17 image data indicating a warning (for example, the warning display 35 illustrated in FIG. 6), while in the case of an audio notification, the notification control unit 10e generates and outputs to the audio controller 18 an alarm sound. Also, in the case of a vibration notification, the notification control unit 10e generates and outputs to a vibration unit (not illustrated) provided in the HMD 1 a control signal for driving the vibration unit.

### (Image capture unit)

The image capture unit 3 includes a lens subsystem made up of the image capture lens 3a, a diaphragm, a zoom lens, a focus lens, and the like, a driving subsystem that causes the lens subsystem to conduct focus operations and zoom operations, a solid-state image sensor array that generates an image capture signal by photoelectric conversion of captured light obtained with the lens subsystem, and the like. The solid-state image sensor array may be realized by a charge-coupled device (CCD) sensor array or a complementary metal-oxide-semiconductor (CMOS) sensor array, for example.

### (Image capture controller)

The image capture controller 11 controls operations of the image capture unit 3 and the image capture signal processor 12 on the basis of instructions from the main controller 10. For example, the image capture controller 11 controls the switching on/off of the operations of the image capture unit 3 and the image capture signal processor 12. The image capture controller 11 is also configured to apply control (motor control) causing the image capture unit 3 to execute operations such as autofocus, automatic exposure adjustment, diaphragm adjustment, and zooming. The image capture controller 11 is also equipped with a timing generator, and controls signal processing operations with timing signals generated by the timing generator for the solid-state image sensors as well as the sample and hold/AGC circuit and video A/D converter of the image capture signal processor 12. In addition, this timing control enables variable control of the image capture frame rate.

Furthermore, the image capture controller 11 controls image capture sensitivity and signal processing in the solid-state image sensors and the image capture signal processor 12. For example, as image capture sensitivity control, the image capture controller 11 is able to conduct gain control of signals read out from the solid-state image sensors, set the black level, control various coefficients for image capture signal processing at the digital data stage, control the correction magnitude in a shake correction process, and the like.

### (Image capture signal processor)

The image capture signal processor 12 is equipped with a sample and hold/automatic gain control (AGC) circuit that applies gain control and waveform shaping to signals obtained by the solid-state image sensors of the image capture unit 3, and a video analog/digital (A/D) converter. Thus, the image capture signal processor 12 obtains an image capture signal as digital data. The image capture signal processor 12 also conducts white balance processing, luma processing, chrome signal processing, shake correction processing, and the like on an image capture signal.

### (Captured image analyzer)

The captured image analyzer 13 is an example of a configuration for acquiring external information. Specifically, the captured image analyzer 13 analyzes image data (a captured image) that has been captured by the image capture unit 3 and processed by the image capture signal processor 12, and obtains information on an image included in the image data.

Specifically, the captured image analyzer 13 conducts analysis such as point detection, line/edge detection, and area segmentation on image data, for example, and outputs analysis results to the used quantity estimation unit 10a and the cooking method identification unit 10b of the main controller 10.

### (Illumination unit, illumination controller)

The illumination unit 4 includes the light emitter 4a illustrated in FIG. 1 and a light emission circuit that causes the light emitter 4a (an LED, for example) to emit light. The illumination controller 14 causes the illumination unit 4 to execute light-emitting operations, according to control by the main controller 10. By attaching the light emitter 4a in the illumination unit 4 as a unit that provides illumination in front as illustrated in FIG. 1, the illumination unit 4 conducts illumination operations in the direction of a user's line of sight.

### (Audio input unit, audio signal processor)

The audio input unit 6 includes the microphones 6a and 6b illustrated in FIG. 1, as well as a mic amp unit and A/D converter that amplifies and processes an audio signal obtained by the microphones 6a and 6b, and outputs audio data to the audio signal processor 15. The audio signal processor 15 conducts processing such as noise removal and source separation on audio data obtained by the audio input unit 6. Processed audio data is then supplied to the main controller 10. Equipping an HMD 1 according to the present embodiment with the audio input unit 6 and the audio signal processor 15 enables voice input from the user, for example.

### (Display controller)

The display controller 17, according to control from the main controller 10, conducts driving control for displaying image data supplied from the notification control unit 10e on the display units 2. The display controller 17 may be made up of a pixel driving circuit for causing display in display units 2 realized as liquid crystal displays, for example. The display controller 17 is also able to control the transparency of each pixel of the display units 2, and put the display units 2 in a see-through state (transparent state or semi-transparent state). In addition, the display controller 17 may include components such as a video processor, a digital signal processor, and a D/A converter, for example, and may conduct luma level adjustment, color correction, contrast adjustment, sharpness (edge enhancement) adjustment, and the like on image data to display.

### (Display units)

The display units 2 display image data under control by the display controller 17. Specifically, the display units 2 according to the present embodiment present a cooking indices display, a warning display, and the like.

### (Audio controller)

The audio controller 18, according to control from the main controller 10, applies control to output audio signal data supplied from the notification control unit 10e from the audio output unit 5. In addition, the audio controller 18 conducts volume adjustment, sound quality adjustment, acoustic effects, and the like on audio signal data to output.

### (Audio output unit)

The audio output unit 5 includes the pair of earphone speakers 5a illustrated in FIG. 1, and an amp circuit for the earphone speakers 5a. Also, the audio output unit 5 made be configured as what is called a bone conduction speaker. The audio output unit 5, according to control from the audio controller 18, outputs (plays back) audio signal data.

### (Storage unit)

The storage unit 22 is a member that records or plays back data with respect to a designated recording medium. The storage unit 22 is realized by a hard disk drive (HDD), for example. Obviously, various media such as flash memory or other solid-state memory, a memory card housing solid-state memory, an optical disc, a magneto-optical disc, and holographic memory are conceivable as the recording medium, and it is sufficient to configure the storage unit 22 to be able to execute recording and playback in accordance with the implemented recording medium.

Also, the storage unit 22 according to the present embodiment stores data such as used quantity estimation data used by the used quantity estimation unit 10a, cooking method identification data used by the cooking method identification unit 10b, and index computation data and index conversion data used by the index computation unit 10c. Also, the storage unit 22 stores information about the user 8, such as medical information, health information, genetic information, and predisposition information.

Furthermore, the storage unit 22 stores cooking indices computed/converted by the index computation unit 10c. In this way, by accumulating cooking indices computed during the cooking process, the HMD 1 is able to generate more accurate cooking indices for the finished dish. In the storage unit 22, the finished dish and its cooking indices (monitor results) are stored in association with each other.

### (Communication unit)

The communication unit 21 sends and receives data to and from external equipment. The communication unit 21 communicates wirelessly with external equipment directly or via a network access point, according to a scheme such as a wireless local area network (LAN), Wi-Fi (Wireless Fidelity, registered trademark), infrared communication, or Bluetooth (registered trademark).

The communication unit 21 according to the present embodiment receives measurement data from a cooking utensil provided with a concentration sensor, for example.

The above thus describes in detail an internal configuration of an HMD 1 according to the present embodiment. Note that although the audio output unit 5, audio input unit 6, audio signal processor 15, and audio controller 18 are illustrated as an audio-related configuration, it is not strictly necessary to provide all of the above. Also, although the communication unit 21 is illustrated as part of the configuration of the HMD 1, it is not strictly necessary to provide the communication unit 21.

According to the above configuration, during the cooking process, the HMD 1 according to the present embodiment is able to estimate the quantities of ingredients to be cooked or seasonings used in cooking, compute cooking indices of the ingredients or seasonings on the basis of the estimation results, and notify the user of the computed cooking indices in real-time. In addition, by accumulating cooking indices computed during the cooking process, the HMD 1 is able to generate more accurate cooking indices for the finished dish. Next, an operational process of an HMD 1 according to the present embodiment will be described.

### <2-2. Operational process of HMD>

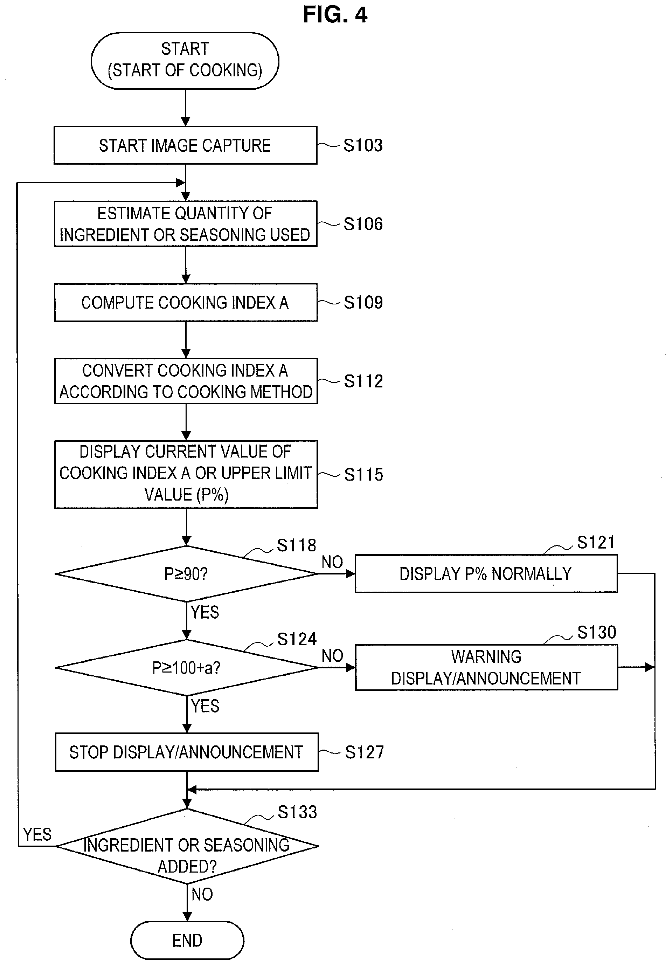

As discussed above, the HMD 1 according to the present embodiment is worn by the user 8, and notifies the user of cooking indices in real-time while the user 8 is cooking. A cooking indices notification by such an HMD 1 will be specifically described hereinafter with reference to FIG. 4.

FIG. 4 is a flowchart illustrating a cooking indices display process by the HMD 1 according to the present embodiment. As illustrated in FIG. 4, first, in step S103 the HMD 1 starts imaging the cooking process with the image capture unit 3.

Next, in step S106, the used quantity estimation unit 10a of the HMD 1 estimates the quantities of ingredients to be cooked or seasonings used in cooking, on the basis of a captured image of the cooking process captured by the image capture unit 3.

Subsequently, in step S109, the index computation unit 10c generates a cooking index A of an ingredient or seasoning, according to a quantity of ingredient or seasoning used that was estimated by the used quantity estimation unit 10a.

Next, in step S112, if a cooking method is identified by the cooking method identification unit 10b, the index computation unit 10c converts the computed cooking index A according to the cooking method.

Subsequently, in step S115, the notification control unit 10e instructs the display controller 17 to display on the display units 2 the cooking index supplied from the index computation unit 10c (the current value of the cooking index A). Alternatively, if the cooking index A and a prescribed limit index (upper limit value) are compared by the index comparison unit 10d, the notification control unit 10e may instruct the display controller 17 to display on the display units 2 the comparison result (the ratio of the current value of the cooking index A versus the upper limit value; P%).

Next, in step S118, the notification control unit 10e judges whether or not the above P% (the ratio of the current value of the cooking index A versus the upper limit value) is 90% or greater.

In the case of being below 90% (S118/No), in step S121 the notification control unit 10e applies control to display P% normally.

On the other hand, in the case of being 90% or greater (S118/Yes), in step S124 the notification control unit 10e judges whether or not P% is 100+a (alpha)% or greater. In other words, the notification control unit 10e judges whether or not the current value of the cooking index A has exceeded the upper limit value+a (alpha).

In the case of being below 100+a% (S124/No), in step S130 the notification control unit 10e instructs the display controller 17 or the audio controller 18 to produce a warning display from the display units 2 or a warning announcement from the audio output unit 5. Thus, the HMD 1 is able to warn the user when the current value of the cooking index A is between 90% and 100+a%.

On the other hand, in the case of 100+a% or greater (S124/Yes), in step S127 the notification controller 10e instructs the display controller 17 or the audio controller 18 to produce a stop display from the display units 2 or a stop announcement from the audio output unit 5.

A stop notification (prohibition notification) has a higher alert level than a warning notification. For example, the notification control unit 10e may cause the display units 2 to display "DO NOT ADD" in large letters, or cause the audio output unit 5 to output a warning sound until the user stops adding the ingredient or seasoning (until the ingredient or seasoning is removed from the angle of view of the image capture unit 3). In addition, if the lid of a seasoning container is motorized, the notification control unit 10e may also transmit a command from the HMD 1 to the seasoning container causing the lid to close, thereby closing the lid of the seasoning container automatically, and preventing the seasoning from being added to the dish.

Subsequently, in step S133, the main controller 10 judges whether or not an ingredient or seasoning has been added. For example, if an action of the user being about to add an ingredient or seasoning to a cooking utensil such as a frying pan is extracted on the basis of a captured image captured by the image capture lens 3a, it is judged that an ingredient or seasoning is added.

If an ingredient or seasoning is added (S133/Yes), in step S106 the used quantity estimation unit 10a estimates the quantity of the added ingredient or seasoning, and in the following step S109, the index computation unit 10c generates a cooking index A of the ingredient or seasoning. At this point, the index computation unit 10c generates the current value of the cooking index A by adding the total value of the currently computed cooking index to the cumulative (total) cooking index since cooking started. Thereafter, the above is repeated from S112 to S133.

If an ingredient or seasoning is not added (S133/No), it is judged that cooking is finished, and the cooking index notification process ends.

The above thus specifically describes a cooking indices display process according to the present embodiment.

### «3. Screen display examples»

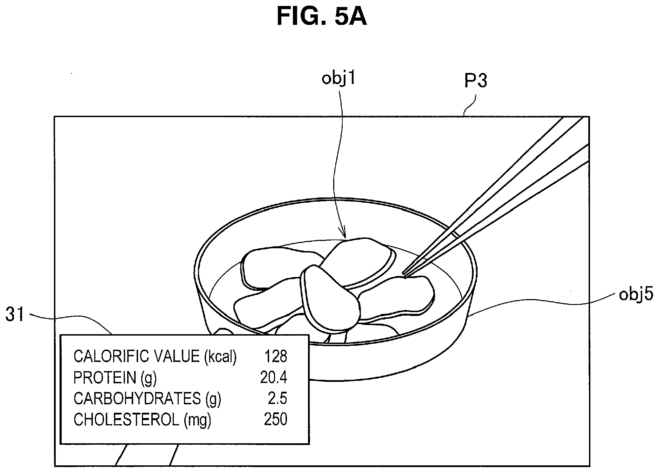

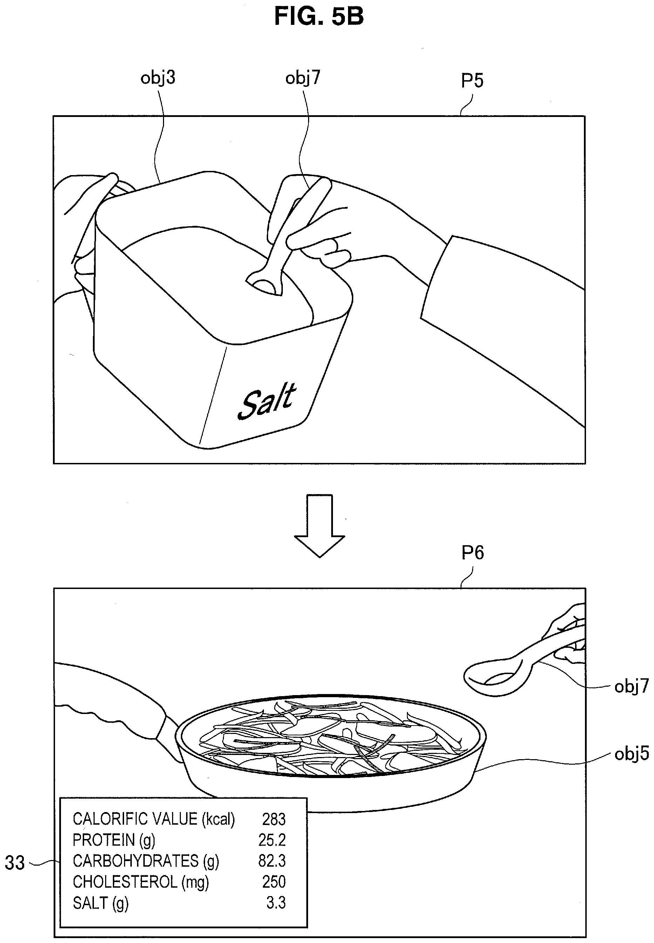

Next, screen display examples of cooking indices according to the present embodiment will be described with reference to FIGS. 5 to 7. As discussed above, the HMD 1 according to the present embodiment is able to display cooking indices of ingredients and seasonings in real-time during the cooking process, and thereby support the dietary lifestyle of the user 8.

### <3-1. Index display>

FIG. 5A is a diagram illustrating an example screen display of cooking indices in the case of adding an ingredient. FIG. 5A illustrates a cooking indices display 31 displayed when a first ingredient obj1 (pork liver) that has been extracted and recognized from the captured image P1 illustrated in FIG. 3 is added to a frying pan obj5 which is an object extracted from the captured image P3, on the basis of an image analysis result for the captured image P3. The HMD 1 according to the present embodiment continually captures the cooking process from the start of cooking (that is, captures a series of cooking operations), and stores features such as the color, shape, and size of each object such as ingredients, seasoning containers, and cooking utensils extracted from a captured image, enabling tracking. Consequently, the HMD 1 is able to continually observe which ingredients and seasonings the user 8 adds (puts in) during the cooking process.

The cooking indices display 31 illustrated in FIG. 5A includes a calorie display and a nutritional components display for the first ingredient obj1 (pork liver). The first ingredient obj1 is estimated to be "pork liver, 100 g" by character recognition of the label from the captured image P1 illustrated in FIG. 3. Consequently, the index computation unit 10c is able to compute cooking indices indicating the calories and nutritional components of "pork liver, 100 g".

Subsequently, as cooking proceeds and other ingredients are added to the frying pan, the index computation unit 10c computes cooking indices for each ingredient, and totals the computed dish indices.

Next, the display of cooking indices for a seasoning will be described with reference to FIG. 5B. FIG. 5B is a diagram illustrating an example screen display of cooking indices in the case of adding a seasoning. As illustrated in the upper part of FIG. 5B, during the cooking process, if an image is captured as the user 8 is scooping up salt with a measuring spoon from a seasoning container of salt, for example, the used quantity estimation unit 10a recognizes the seasoning container obj3 and the measuring spoon obj7 on the basis of image analysis results for the captured image P5 that was captured. Since the seasoning container obj3 is the "Salt" seasoning container obj3 already extracted and recognized from the captured image P1 illustrated in FIG. 3, the used quantity estimation unit 10a is able to estimate the quantity of salt used according to how much is scooped up with the measuring spoon obj7. Note that the used quantity estimation of a seasoning is not limited to such image analysis, and if the measuring spoon is provided with a salt concentration sensor, for example, the used quantity estimation unit 10a may also estimate the quantity of salt used on the basis of estimation data transmitted from the measuring spoon.

Next, if an action of being about to add the salt scooped up with the measuring spoon obj7 to the frying pan obj5 is extracted on the basis of image analysis results for the captured image P6 illustrated in the lower part of FIG. 5B, the index computation unit 10c computes cooking indices of the added seasoning (herein, "salt").

Subsequently, the notification control unit 10e instructs the display controller 17 to display on the display units 2 a cooking indices display 33 including a display of the computed cooking indices.

As described above, the HMD 1 according to the present embodiment is able to continually observe the cooking process, compute cooking indices for ingredients to be cooked and seasonings used in the cooking, and notify the user 8. Note that a notification by the HMD 1 is not limited to a notification of computed cooking indices, and may also be a warning notification or a comparison result notification based on comparison results from comparing the computed cooking indices to prescribed values. Hereinafter, as an example, a screen display based on a comparison result of a cooking index and a prescribed value will be described with reference to FIGS. 6 and 7.

### <3-2. Display based on comparison result>

### (3-2-1. Warning display)

FIG. 6 is a diagram illustrating an example image display of a warning display according to the present embodiment. On the basis of an image analysis result for the captured image P7 illustrated in FIG. 6, the index computation unit 10c computes a cooking index indicating the quantity of salt added to the frying pan obj5 with the measuring spoon obj7, and supplies the computed cooking index to the index comparison unit 10d. At this point, the index computation unit 10c may also compute a cumulative cooking index by adding the cooking index indicating the quantity of added salt to a cooking index indicating the total quantity of used salt accumulated during the cooking process.

Subsequently, the index comparison unit 10d compares the cooking index computed by the index computation unit 10c to a prescribed value. The prescribed value to compare against is a prescribed upper limit value prioritizing health, or a prescribed upper limit value prioritizing taste preferences, for example, as discussed earlier.

Subsequently, on the basis of a comparison result supplied from the index comparison unit 10d, if the cooking index indicating the quantity of salt computed by the index computation unit 10c approaches the prescribed value, the notification control unit 10e causes the display units 2 to display a warning display 35 indicating a warning of "The amount of salt is over the limit!", as illustrated in FIG. 6. Herein, approaching the prescribed value refers to a case in which the quantity of salt indicated by the cooking index is a ratio from 90% to 100+a% versus the upper limit value, for example.

Also, if the cooking index approaches and then exceeds the prescribed value, and specifically reaches a ratio equal to or greater than 100+a% versus the upper limit value, the notification control unit 10e may also cause the display units 2 to display a prohibition display indicating a warning of "NO MORE SALT ALLOWED".

### (3-2-2. Comparison result display)

As described above, when a prescribed cooking index approaches a prescribed value, the notification control unit 10e is able to present a warning display and warn the user 8. However, some cases are anticipated in which, even though the user may be notified that the salt content or sugar content exceeds an upper limit value, not adding an ingredient is undesirable in terms of the completed dish, such as when adding an important ingredient last to finish cooking. Consequently, the index comparison unit 10d of the HMD 1 according to the present embodiment is also able to compare a cooking index computed by the index computation unit 10c to a limit index (upper limit value) for when cooking is complete, and by notifying the user 8 of the comparison result with the notification control unit 10e, is able to present a notification that accounts for the upper limit value at the time of completion. Hereinafter, an example of such a notification of a comparison result will be described with reference to FIG. 7.

FIG. 7 is a diagram illustrating an example screen display of a comparison result notification according to the present embodiment. As illustrated in FIG. 7, on the basis of a comparison result by the index comparison unit 10d, the notification control unit 10e instructs the display controller 17 to cause the display units 2 to display a bar display 37 indicating the ratio of the current cooking index versus the upper limit value for when cooking is complete. In the example illustrated in FIG. 7, the bar display 37 is superimposed onto the captured image P9, but the display controller 17 may also control the transmittance of the display units 2 and present an AR display of the bar display 37.

Consequently, the user 8 is able to grasp the current ratio of the cooking index, and make sure to not add too much of a specific seasoning or ingredient while cooking. Note that in the example illustrated in FIG. 7, the bar display 37 is superimposed onto the captured image P9, but the display controller 17 may also control the transmittance of the display units 2 and present an AR display of the bar display 37.

### «4. Supplementary remarks»

As discussed earlier, during the cooking process, the HMD 1 according to the present embodiment is able to compute cooking indices for ingredients to be cooked and seasonings used in the cooking, and notify the user 8 of the computed cooking indices in real-time. In addition, by continually accumulating the computed cooking indices, the HMD 1 is able to generate more accurate cooking indices for the completed dish. At this point, usage examples of cooking indices generated in this way will be described as supplemental remarks.

Cooking indices for a dish are used to notify the user of the calories and nutritional components of a dish when eating and drinking, for example. By notifying the user of the calories and nutritional components of a dish when eating and drinking, the user is able to intuitively grasp the calories and nutritional components consumed.

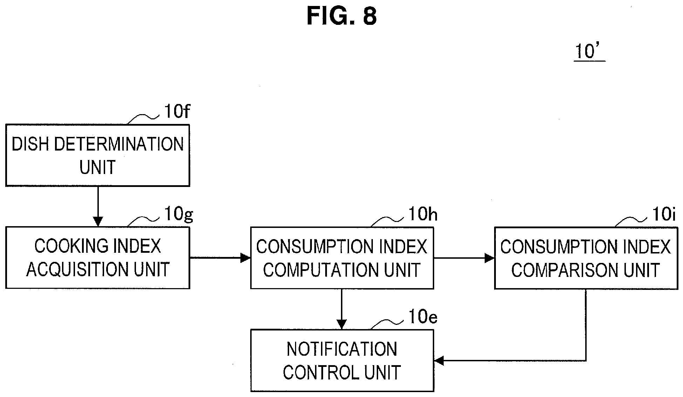

Such a notification of calories and nutritional components when eating and drinking may also be conducted by the notification control unit 10e of the HMD 1. Specifically, by including a main controller 10' illustrated in FIG. 8, the HMD 1 is able to use cooking indices generated during the cooking process to notify the user 8 of the cooking indices of a dish when eating or drinking.

### <4-1. Functional configuration of main controller>

As illustrated in FIG. 8, the main controller 10' functions as a dish determination unit 10f, a cooking index acquisition unit 10g, a consumption index computation unit 10h, a consumption index comparison unit 10i, and a notification control unit 10e. The dish determination unit 10f determines the dishes and number of dishes on the basis of analysis results (features such as the color, shape, and size of a dish) for a captured image, captured by the image capture unit 3, of a dish to be consumed. The dish determination method by the dish determination unit 10f may not only be based on captured image analysis results, but also be a determination method based on a scan result of an IC tag, marker, or the like attached to a plate on which the dish is served.

The cooking index acquisition unit 10g acquires cooking indices of a dish determined by the dish determination unit 10f. At this point, suppose a case in which cooking indices computed by the index computation unit 10c illustrated in FIG. 2 during the cooking process are accumulated in the storage unit 22, and stored in a database in association with images of completed dishes and names of dishes. Consequently, the cooking index acquisition unit 10g is able to acquire cooking indices from the storage unit 22.

The consumption index computation unit 10h computes indices of food and drink consumed by the user 8. Specifically, the consumption index computation unit 10h computes indices of the portion of food or drink consumed by the user 8, on the basis of an analysis result of a captured image that is captured by the image capture unit 3. Indices of food or drink consumed refer to indicates that indicate intake calories in other words, and the masses of nutritional components that are taken in.

The consumption index comparison unit 10i compares a consumption index computed by the consumption index computation unit 10h to a prescribed value. The prescribed value refers to an upper limit value on calorie intake in one day, an upper limit value on the intake of a nutritional component in one day, or the like, for example.

The notification control unit 10e instructs the display controller 17 or the audio controller 18 to notify the user 8 of consumption indices computed by the consumption index computation unit 10h and comparison results from the consumption index comparison unit 10i.

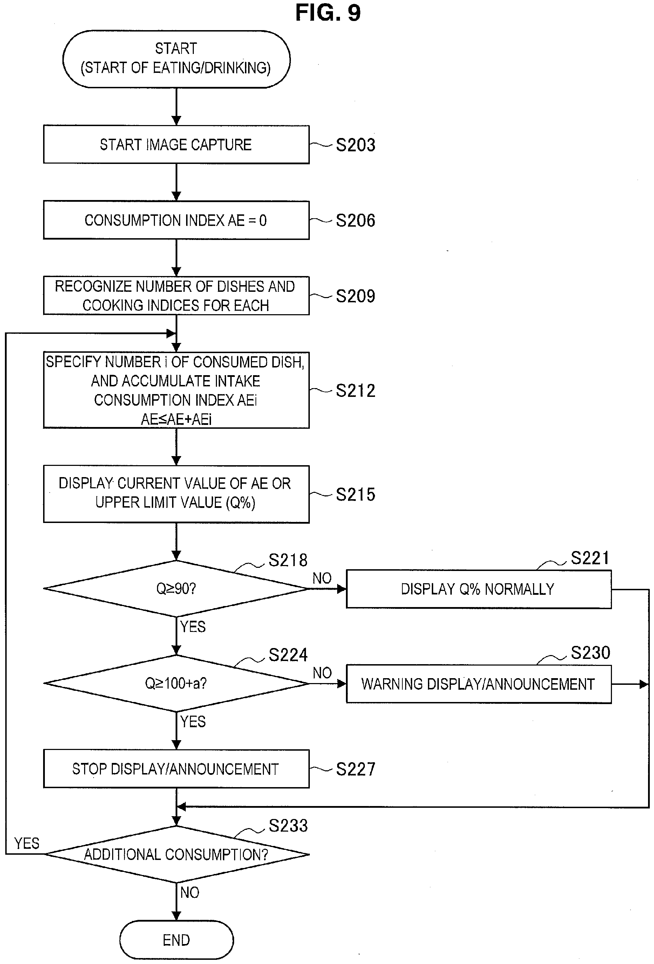

The above thus specifically described a functional configuration of the main controller 10' of the HMD 1. Next, an operational process for the case of notifying a user of the calories and nutritional components of a dish when eating and drinking on the basis of the cooking indices of the dish will be described with reference to FIG. 9.

### <4-2. Consumption indices notification process>

FIG. 9 is a flowchart illustrating an operational process when using cooking indices to notify a user of consumption indices while eating and drinking. As illustrated in FIG. 9, first, in step S203 the HMD 1 starts capturing images of eating and drinking with the image capture unit 3. Herein, suppose that a consumption index AE is a quantity consumed by the user 8. The consumption index AE indicates intake calories or the mass of a nutritional component taken in.

Next, in step S206, the main controller 10' of the HMD 1 recognizes that the consumption index AE = 0 at the start of eating and drinking.

Next, in step S209, the dish determination unit 10f of the main controller 10' determines the number of dishes on the basis of an analysis result of a captured image that is captured by the image capture unit 3, and the cooking index acquisition unit 10g acquires a cooking index A of each dish. The dish determination unit 10f may also assign a number to each dish when determining the number of dishes. Also, in the case of five dishes numbered from 1 to 5, for example, the cooking index acquisition unit 10g acquires cooking indices A1 to A5 respectively for each dish.

Next, in step S212, the consumption index computation unit 10h specifies the number i of the dish that the user 8 is currently consuming, on the basis of an analysis result of a captured image that is captured by the image capture unit 3, and totals the cooking index Ai of the corresponding dish as the intake consumption index AEi. In other words, the consumption index computation unit 10h computes the consumption index AE (current value) as being equal to AEi (the consumption index just consumed). In addition, when the intake consumption index AEi is additional consumption, the consumption index computation unit 10h computes the consumption index AE (current value) as being equal to AE (the previous total of the consumption index) plus AEi (the consumption index just consumed).

Next, in step S215, the notification control unit 10e of the main controller 10 instructs the display controller 17 to display on the display units 2 the current value of the consumption index AE computed by the consumption index computation unit 10h. Alternatively, if the current consumption index AE and a prescribed value (intake upper limit value) are compared by the consumption index comparison unit 10i, the notification control unit 10e may instruct the display controller 17 to display on the display units 2 the comparison result (the ratio of the current value of the consumption index AE versus the upper limit value; Q%). The intake upper limit value is an upper limit value on calorie intake in one day, an upper limit value on calorie intake in one week, or an upper limit value on cholesterol in one day, or the like, for example. Such an upper limit value may also be set on the basis of a user's medical information and health information.

Next, in step S218, the notification control unit 10e judges whether or not the above Q% (the ratio of the current value of the consumption index AE versus the intake upper limit value) is 90% or greater.

In the case of being below 90% (S218/No), in step S221 the notification control unit 10e applies control to display Q% normally.

On the other hand, in the case of being 90% or greater (S218/Yes), in step S224 the notification control unit 10e judges whether or not Q% is 100+a (alpha)% or greater. In other words, the notification control unit 10e judges whether or not the current value of the consumption index AE has exceeded the intake upper limit value+a (alpha).

In the case of being below 100+a% (S224/No), in step S230 the notification controller 10e instructs the display controller 17 or the audio controller 18 to produce a warning display from the display units 2 or a warning announcement from the audio output unit 5. Thus, in the case where the current value of the consumption index AE is between 90% and 100+a%, the HMD 1 issues a warning to the user.

On the other hand, in the case of 100+a% or greater (S224/Yes), in step S227 the notification controller 10e instructs the display controller 17 or the audio controller 18 to produce a stop display from the display units 2 or a stop announcement from the audio output unit 5.

A stop notification (prohibition notification) has a higher alert level than a warning notification. For example, the notification controller 10e may cause the display units 2 to display "STOP EATING" in large letters, or cause the audio output unit 5 to output a warning sound until the user stops eating.

Subsequently, in step S233, the main controller 10' judges whether or not there is additional consumption. For example, if an action of the user being about to continue eating or drinking is extracted on the basis of a captured image captured by the image capture lens 3a, it is judged that there is additional consumption.

If there is additional consumption (S233/Yes), in step S212 the consumption index computation unit 10h computes, on the basis of the cooking index Ai of the dish i being additionally consumed, the consumption index AE (current value) as being equal to AE (the previous total of the consumption index) plus AEi (the consumption index just consumed). Thereafter, the above is repeated from S215 to S233.

If there is no additional consumption (S233/No), it is judged that eating and drinking is finished, and the consumption index notification process ends.

### «5. Conclusion»

As discussed above, with an HMD 1 according to the present embodiment, it is possible to compute indices during the cooking process, and notify the user of the cooking indices in real-time while cooking. In addition, cooking indices may be converted according to the cooking method for the dish.

Also, the HMD 1 is able to present a warning notification or a prohibition notification based on a comparison result from comparing a current cooking index to a prescribed value, and notify the user of the ratio of the current value of the cooking index versus the prescribed value or the like.

Furthermore, since the HMD 1 continually observes the cooking process, computes cooking indices of ingredients to be cooked and seasonings used in the cooking, and accumulates the computed cooking indices, the HMD 1 is able to generate more accurate cooking indices for the finished dish.

The foregoing thus describes preferred embodiments of the present technology in detail and with reference to the attached drawings. However, the present disclosure is not limited to such examples. It is clear to persons ordinarily skilled in the technical field of the present disclosure that various modifications or alterations may occur insofar as they are within the scope of the technical ideas stated in the claims, and it is to be understood that such modifications or alterations obviously belong to the technical scope of the present disclosure.

For example, it is possible to create a computer program for causing hardware such as a CPU, ROM, and RAM built into the HMD 1 to exhibit the functionality of the HMD 1 discussed earlier. A computer-readable storage medium made to store such a computer program is also provided.

Also, in the above respective embodiments, although an HMD 1 is used as an example of an information processing device, an information processing device according to the present embodiment is not limited to an HMD 1, and may also be a display control system formed from a smartphone and an eyeglasses-style display, for example. The smartphone (information processing device) is connectable to the eyeglasses-style display in a wired or wireless manner, and is able to transmit and receive data.

Herein, the eyeglasses-style display includes a wearing unit having a frame structure that wraps halfway around the back of the head from either side of the head, and is worn by a user by being placed on the pinna of either ear, similarly to the HMD 1 illustrated in FIG. 1. Also, the eyeglasses-style display is configured such that, in the worn state, a pair of display units for the left eye and the right eye are placed immediately in front of either eye of the user, or in other words at the locations where the lenses of ordinary eyeglasses are positioned. By controlling the transmittance of the liquid crystal panels of the display units 2, the HMD 1 is able to set a see-through state, or in other words a transparent or semi-transparent state, and thus ordinary activities are not impaired even if the user wears the HMD 1 continuously like eyeglasses.

Also, the eyeglasses-style display is provided with an image capture lens for capturing the user's gaze direction while in the worn state, similarly to the HMD 1 illustrated in FIG. 1. The eyeglasses-style display transmits a captured image to the smartphone (information processing device).

The smartphone (information processing device) includes functions similar to the main controller 10, and estimates quantities of ingredients to be cooked and seasonings used from a captured image, and generates a cooking indices display image indicating the estimated cooking indices. Additionally, the smartphone (information processing device) transmits a generated cooking indices display image to the eyeglasses-style display, and the cooking indices display image is displayed on the display units of the eyeglasses-style display.

Application is also conceivable to an eyeglasses-style device that, although similar in shape to an eyeglasses-style display, does not include display functions. In this case, the cooking process is captured by a camera, provided on the eyeglasses-style device, that captures the wearer's (the user's) gaze direction, and a captured image is transmitted to the smartphone (information processing device). Subsequently, the smartphone (information processing device) generates a cooking indices display image indicating cooking indices on the basis of the cooking process depicted in the captured image, which is displayed on a display of the smartphone.

Furthermore, although the foregoing embodiments described the used quantity estimation unit 10a determining quantities of ingredients and seasonings used and the cooking method identification unit 10b identifying the cooking method on the basis of a captured image analysis result from the captured image analyzer 13 of the HMD 1, such a captured image analyzing process may also be conducted in the cloud. The HMD 1 sends a captured image of the cooking process to the cloud via the communication unit 21, receives a result that has been analyzed in the cloud (on an analysis server, for example), and on the basis thereof, conducts used quantity estimation and cooking method identification with the used quantity estimation unit 10a and the cooking method identification unit 10b.

### Reference Signs List

- 1: head-mounted display (HMD)
- 2: display unit
- 3: image capture unit
- 3a: image capture lens
- 4: illumination unit
- 4a: light emitter
- 5: audio output unit
- 6: audio input unit
- 10, 10': main controller
- 10a: used quantity estimation unit
- 10b: cooking method identification unit
- 10c: index computation unit
- 10d: index comparison unit
- 10e: notification control unit
- 10f: dish determination unit
- 10g: cooking index acquisition unit
- 10h: consumption index computation unit
- 10i: consumption index comparison unit
- 11: image capture controller
- 12: image capture signal processor
- 13: captured image analyzer
- 14: illumination controller
- 15: audio signal processor
- 16: output data processor
- 17: display controller
- 18: audio controller
- 21: communication unit
- 22: storage unit

## Claims

1. An information processing device **characterized in** comprising:
an estimation unit configured to estimate a used quantity of at least one of an ingredient to be cooked and a seasoning used in cooking, on the basis of a signal detected by a sensor in real-time every time an ingredient or seasoning is added during a cooking process;
an index computation unit configured to compute a cooking index indicating a value of mass and/or a calorific value of the ingredient or the seasoning according to the estimated quantity of the ingredient or the seasoning, by accumulating cooking indices computed since the cooking process started to a cumulative cooking index; and
a notification control unit configured to perform control to issue a notification of the cooking index computed by the index computation unit.

2. The information processing device according to claim 1, further comprising
a cooking method identification unit configured to identify a cooking method on the basis of a captured image acquired by an image capture sensor or on the basis of measurement data detected by sensors selected from an infrared sensor, a temperature sensor and a humidity sensor,
wherein the index computation unit converts the computed cooking index according to the cooking method identified by the cooking method identification unit by using a database containing cooking index conversion data that associate cooking methods with changes in respective cooking indices.

3. The information processing device according to claim 1, further comprising
a display unit configured to display the cooking index according to the control by the notification control unit.

4. The information processing device according to claim 1, wherein
when the cooking index computed by the index computation unit approaches a prescribed value, the notification control unit performs control to present a warning notification to a user.

5. The information processing device according to claim 4, wherein
the prescribed value is a limit index, and
the cumulative cooking index is compared to a plurality of limit indices.

6. The information processing device according to claim 5, wherein
the plurality of limit indices include a prescribed value prioritizing health and a prescribed value prioritizing taste.

7. The information processing device according to claim 1, wherein
the notification control unit performs control to present a warning notification to a user according to a comparison result of the cooking index accumulated by the index computation unit and a prescribed value for when cooking is complete.

8. The information processing device according to claim 1, wherein
the value of mass or the calorific value is a value of mass or calorific value per nutritional component.

9. The information processing device according to claim 1, wherein
the sensor is a concentration sensor, an imaging sensor, or a smell sensor.

10. The information processing device according to claim 1, wherein
the information processing device is a head-mounted-style or eyeglasses-style head-mounted display.

11. The information processing device according to claim 1, wherein
the information processing device is a smartphone, a mobile phone, a tablet, a PC, a digital camera, or a digital camcorder.

12. A storage medium **characterized in** having a program stored therein, the program causing a computer to function as:
an estimation unit configured to estimate a used quantity of at least one of an ingredient to be cooked and a seasoning used in cooking, on the basis of a signal detected by a sensor in real-time every time an ingredient or seasoning is added during a cooking process;
an index computation unit configured to compute a cooking index indicating a value of mass and/or a calorific value of the ingredient or the seasoning according to the estimated quantity of the ingredient or the seasoning, by accumulating cooking indices computed since the cooking process started to a cumulative cooking index; and
a notification control unit configured to perform control to issue a notification of the cooking index computed by the index computation unit.

## Patentansprüche

1. Datenverarbeitungsvorrichtung, **dadurch gekennzeichnet, dass** sie Folgendes umfasst:
eine Schätzeinheit, die konfiguriert ist, jedes Mal, wenn während eines Kochprozesses eine Zutat oder eine Würze zugegeben wird, auf der Grundlage eines durch einen Sensor in Echtzeit detektieren Signals eine verwendete Menge einer zu kochenden Zutat und/oder einer beim Kochen verwendeten Würze zu schätzen;
eine Indexberechnungseinheit, die konfiguriert ist, in Übereinstimmung mit der geschätzten Menge der Zutat oder der Würze durch Summieren von Kochindizes, die berechnet wurden, seit der Kochprozess begonnen wurde, zu einem kumulativen Kochindex einen Kochindex zu berechnen, der einen Wert der Masse und/oder einen Brennwert der Zutat oder der Würze angibt; und
eine Meldungssteuereinheit, die konfiguriert ist, eine Steuerung zum Ausgeben einer Meldung des durch die Indexberechnungseinheit berechneten Kochindex auszuführen.

2. Datenverarbeitungsvorrichtung nach Anspruch 1, die ferner Folgendes umfasst:
eine Kochverfahren-Identifizierungseinheit, die konfiguriert ist, auf der Grundlage eines aufgenommenen Bilds, das durch den Bildaufnahmesensor erfasst wird, oder auf der Grundlage von Messdaten, die durch Sensoren detektiert werden, die aus einem Infrarotsensor, aus einem Temperatursensor und aus einem Feuchtesensor ausgewählt sind, ein Kochverfahren zu identifizieren,
wobei die Indexberechnungseinheit den berechneten Kochindex unter Verwendung einer Datenbank, die Kochindexumsetzungsdaten enthält, die Kochverfahren zu Änderungen jeweiliger Kochindizes zuordnen, in Übereinstimmung mit dem durch die Kochverfahren-Identifizierungseinheit identifizierten Kochverfahren umsetzt.

3. Datenverarbeitungsvorrichtung nach Anspruch 1, die ferner Folgendes umfasst:
eine Anzeigeeinheit, die konfiguriert ist, den Kochindex in Übereinstimmung mit der Steuerung durch die Meldungssteuereinheit anzuzeigen.

4. Datenverarbeitungsvorrichtung nach Anspruch 1, wobei
die Meldungssteuereinheit eine Steuerung zum Darstellen einer Warnungsmeldung für einen Benutzer ausführt, wenn sich der durch die Indexberechnungseinheit berechnete Kochindex einem vorgeschriebenen Wert annähert.

5. Datenverarbeitungsvorrichtung nach Anspruch 4, wobei
der vorgeschriebene Wert ein Grenzindex ist, und der kumulative Kochindex mit mehreren Grenzindizes verglichen wird.

6. Datenverarbeitungsvorrichtung nach Anspruch 5, wobei
die mehreren Grenzindizes einen vorgeschriebenen Wert, der die Gesundheit priorisiert, und einen vorgeschriebenen Wert, der den Geschmack priorisiert, enthalten.

7. Datenverarbeitungsvorrichtung nach Anspruch 1, wobei
die Meldungssteuereinheit die Steuerung zur Darstellung einer Warnungsmeldung für einen Benutzer in Übereinstimmung mit einem Vergleichsergebnis des durch die Indexberechnungseinheit summierten Kochindex und eines vorgeschriebenen Werts dafür, wann das Kochen abgeschlossen ist, ausführt.

8. Datenverarbeitungsvorrichtung nach Anspruch 1, wobei
der Wert der Masse oder der Brennwert ein Wert der Masse oder ein Brennwert pro Nahrungsbestandteil ist.

9. Datenverarbeitungsvorrichtung nach Anspruch 1, wobei
der Sensor ein Konzentrationssensor, ein Bilderzeugungssensor oder ein Geruchssensor ist.

10. Datenverarbeitungsvorrichtung nach Anspruch 1, wobei
die Datenverarbeitungsvorrichtung ein Datenhelm eines am Kopf angebrachten Stils oder eines an der Brille angebrachten Stils ist.

11. Datenverarbeitungsvorrichtung nach Anspruch 1, wobei
die Datenverarbeitungsvorrichtung ein Smartphone, ein Mobiltelefon, ein Tablet, ein PC, eine Digitalkamera oder ein digitaler Camcorder ist.

12. Speichermedium, **dadurch gekennzeichnet, dass** darin ein Programm gespeichert ist, wobei das Programm veranlasst, dass ein Computer als Folgendes fungiert:
eine Schätzeinheit, die konfiguriert ist, jedes Mal, wenn während eines Kochprozesses eine Zutat oder eine Würze zugegeben wird, auf der Grundlage eines durch einen Sensor in Echtzeit detektieren Signals eine verwendete Menge einer zu kochenden Zutat und/oder einer beim Kochen verwendeten Würze zu schätzen;
eine Indexberechnungseinheit, die konfiguriert ist, in Übereinstimmung mit der geschätzten Menge der Zutat oder der Würze durch Summieren von Kochindizes, die berechnet wurden, seit der Kochprozess begonnen wurde, zu einem kumulativen Kochindex einen Kochindex zu berechnen, der einen Wert der Masse und/oder einen Brennwert der Zutat oder der Würze angibt; und
eine Meldungssteuereinheit, die konfiguriert ist, eine Steuerung zum Ausgeben einer Meldung des durch die Indexberechnungseinheit berechneten Kochindex auszuführen.

## Revendications

1. Dispositif de traitement d'informations **caractérisé en ce qu'**il comprend :
une unité d'estimation configurée pour estimer une quantité utilisée d'au moins un élément parmi un ingrédient à faire cuire et un assaisonnement utilisé pour la cuisson, d'après un signal détecté par un capteur en temps réel chaque fois qu'un ingrédient ou un assaisonnement est ajouté au cours d'un processus de cuisson ;
une unité de calcul d'indice configurée pour calculer un indice de cuisson indiquant une valeur de masse et/ou une valeur calorifique de l'ingrédient ou de l'assaisonnement en fonction de la quantité estimée de l'ingrédient ou de l'assaisonnement, en accumulant des indices de cuisson calculés depuis le début du processus de cuisson, jusqu'à atteindre un indice de cuisson cumulatif ; et
une unité de commande de notification configurée pour effectuer une commande pour faire émettre une notification de l'indice de cuisson calculé par l'unité de calcul d'indice.

2. Dispositif de traitement d'informations selon la revendication 1, comprenant également :
une unité d'identification de méthode de cuisson configurée pour identifier une méthode de cuisson à partir de l'image capturée acquise par un capteur de capture d'image ou à partir de données de mesure détectées par des capteurs choisis parmi un capteur infrarouge, un capteur de température et un capteur d'humidité,
dans lequel l'unité de calcul d'indice convertit l'indice de cuisson calculé en fonction de la méthode de cuisson identifiée par l'unité d'identification de méthode de cuisson au moyen d'une base de données contenant des données de conversion d'indice de cuisson qui associent des méthodes de cuisson à des variations dans les indices de cuisson respectifs.

3. Dispositif de traitement d'informations selon la revendication 1, comprenant également :
une unité d'affichage configurée pour afficher l'indice de cuisson en fonction de la commande effectuée par l'unité de commande de notification.

4. Dispositif de traitement d'informations selon la revendication 1, dans lequel
lorsque l'indice de cuisson calculé par l'unité de calcul d'indice approche d'une valeur prescrite, l'unité de commande de notification effectue une commande pour présenter une notification d'avertissement à un utilisateur.

5. Dispositif de traitement d'informations selon la revendication 4, dans lequel
la valeur prescrite est un indice de limite, et
l'indice de cuisson cumulatif est comparé à une pluralité d'indices de limite.

6. Dispositif de traitement d'informations selon la revendication 5, dans lequel
la pluralité d'indices de limite contient une valeur prescrite donnant la priorité à la santé et une valeur prescrite donnant la priorité au goût.

7. Dispositif de traitement d'informations selon la revendication 1, dans lequel
l'unité de commande de notification effectue une commande pour présenter une notification d'avertissement à un utilisateur en fonction d'un résultat de la comparaison de l'indice de cuisson accumulé par l'unité de calcul d'indice à une valeur prescrite, pour indiquer le moment où la cuisson est terminée.

8. Dispositif de traitement d'informations selon la revendication 1, dans lequel
la valeur de masse ou la valeur calorifique est une valeur de masse ou une valeur calorifique par composant nutritionnel.

9. Dispositif de traitement d'informations selon la revendication 1, dans lequel
le capteur est un capteur de concentration, un capteur d'imagerie ou un capteur d'odeur.

10. Dispositif de traitement d'informations selon la revendication 1,
le dispositif de traitement d'informations étant un afficheur de type visiocasque ou de type lunettes.

11. Dispositif de traitement d'informations selon la revendication 1,
le dispositif de traitement d'informations étant un mobile multifonction, un téléphone mobile, une tablette, un PC, une caméra numérique ou un caméscope numérique.

12. Support de stockage **caractérisé en ce qu'**un programme est stocké dessus, le programme amenant un ordinateur à fonctionner en tant que :
unité d'estimation configurée pour estimer une quantité utilisée d'au moins un élément parmi un ingrédient à faire cuire et un assaisonnement utilisé pour la cuisson, d'après un signal détecté par un capteur en temps réel chaque fois qu'un ingrédient ou un assaisonnement est ajouté au cours d'un processus de cuisson ;
unité de calcul d'indice configurée pour calculer un indice de cuisson indiquant une valeur de masse et/ou une valeur calorifique de l'ingrédient ou de l'assaisonnement en fonction de la quantité estimée de l'ingrédient ou de l'assaisonnement, en accumulant des indices de cuisson calculés depuis le début du processus de cuisson, jusqu'à atteindre un indice de cuisson cumulatif ; et
unité de commande de notification configurée pour effectuer une commande pour faire émettre une notification de l'indice de cuisson calculé par l'unité de calcul d'indice.
